# EUROPEAN PATENT SPECIFICATION

(11) **EP 1 915 477 B1**
(45) Date of publication and mention of the grant of the patent: **18.01.2012**
(21) Application number: 06796242.3
(22) Date of filing: 28.07.2006
(51) Int. Cl.: D04H 11/00, B05D 1/14, D06Q 1/14

(54) **METHOD FOR PRODUCING A WEBLIKE PRODUCT FOR THE PRODUCTION OF ABSORBENT ITEMS AND SIMILAR, PRODUCT SO OBTAINED AND ABSORBENT ARTICLE INCLUDING SAID PRODUCT**
VERFAHREN ZUR HERSTELLUNG EINES BAHNFÖRMIGEN PRODUKTS FÜR DIE HERSTELLUNG SAUGFÄHIGER ARTIKEL U.Ä, AUF DIESE WEISE HERGESTELLTES PRODUKT UND SAUGFÄHIGER ARTIKEL MIT DIESEM PRODUKT
PROCEDE DE PRODUCTION D'UN PRODUIT EN BANDE POUR LA CONFECTION D'ARTICLES ABSORBANTS ET SIMILAIRES, PRODUIT AINSI OBTENU, ET ARTICLES ABSORBANTS INCLUANT LEDIT PRODUIT

(30) Priority: 03.08.2005 IT FI20050172
(43) Date of publication of application: 30.04.2008
(73) Proprietor: Fintex and Partners Italia S.p.A., 51100 Pistoia (IT)
(72) Inventor: CECCONI, Riccardo, I-59100 Prato (IT); BULLERI, Barbara, I-51019 Ponte Buggianese, Pistoia (IT); ALLEGRINI, Chiara, I-55100 S. Cassiano A Vico, Lucca (IT)
(74) Representative: Mannucci, Michele
(86) International application number: PCT/IT2006/000590
(87) International publication number: WO 2007/015286

(56) References cited:
- EP-A1- 0 737 462
- EP-A2- 0 246 476
- GB-A- 945 392
- GB-A- 1 306 509
- JP-A- 11 138 672

## Description

### Technical field

This invention deals with improvements in the production of absorbent items, like nappies, sanitary towels, incontinence pads and similar. More specifically, this invention deals with improvements in the production of the components used to coat absorbent and sanitary items externally.

### State of the art

In the production of sanitary towels, nappies and other sanitary items absorbent structures are usually used enclosed between a top sheet, which comes into contact with the epidermis of the user, and a lower back sheet, turned outwards in relation to the skin of who is wearing the absorbent item. The top and back sheets are obtained by cutting a continuous semi-finished product, produced in a preceding phase of production.

The coating layers, and in particular the top sheet must have a surface that is soft to touch. Moreover the back sheet must be impermeable to liquids, while the top sheet must have a structure to allow fast passage of the biological liquid (blood, urine or other) from the external side towards the internal side, that is towards the mass of absorbent material making up the core of the towel.

Usually the back sheet is produced in polymer film, which can be coated in textile material, to provide the required textile feeling to the finished product. The top sheet is often also produced in polymer film or in non-woven fabric. To allow the outflow of liquids, the substrate or support that constitutes the base of the top sheet (be it a film, or non-woven fabric) can have a perforation. The top sheet, above all if produced in polymer film, can also be coated in textile material.

Among the various methods suggested for the surface treatment and perforation of the polymer film, and among the various absorbent items which use polymer film or other coating material in various configurations by way of example those described in patents USA 3,484,835; 3,911,187; 3,950,480; 3,957,414; 4,151,240; 4,342,314; 4,463,045; 3,967,623; 3,665,921; 5,171,238; 3,945,386 are mentioned. Other techniques of perforation are described in WO 93/12749. The publication GB-A-2,171,016 describes a sanitary towel with an external structure characterized by the incorporation of fibers in an external coating layer.

EP-A-737,462 describes a weblike product, i.e. a sheet or ply product, composed of a base formed by a polymer film, on which is evenly distributed a resin or glue, which serves as an anchor to a flock fiber. In an embodiment of this product, it is foreseen that the resin is laid in longitudinal strips, so that bands coated in flock fiber and bands free of fiber can form on the film. The fiber free bands or zones usually correspond to a strip which, in the final absorbent item coated with this material, is arranged in correspondence to the central area where the biological liquid is collected. In this area the base film is also apertured to allow the fluid to pass into the absorbent core. The lateral surface bands of the top sheet are, on the other hand, entirely covered by flock fiber, that is of fiber applied by flocking.

JP-59-222330 describes an absorbent product in which flock fibers of great length are applied on the internal surface of a layer of coating of the absorbent item and form part of the absorbent core. They are not therefore, destined to come into contact with; the body of the user who wears the absorbent item.

Further absorbent items coated in plastic material with a fiber coating applied by flocking are described in patents USA no. 6,497,688 and 6,365,794 and in European patent EP-B-1,070,492.

Flocking is a technique of fiber application in which the film, on which an anchor resin has been applied, is made pass under one or more fiber distribution heads, which fibers have been previously treated to become polarized. The fibers are entered into an electrostatic field, whose lines of force are essentially at right angles to the surface of the polymer film and are thus made fall onto the film itself in an essentially orthogonal way to the surface of the film. They are anchored to the film by the effect of resin reticulation.

The need to pre-treat or, what is technically called, "activate" the fibers to polarize them makes the fibers particularly expensive. They must, in fact, be immersed into a specific saline solution and absorb ionic groups, which distribute themselves appropriately in the fibers. This process of fiber activation directs them according to the lines of force of the electrostatic field in which they are inserted to be distributed on the film.

To reduce the cost of semi-finished products composed of the film coated by flocking, and therefore to reduce the costs of the absorbent items which make use of such a semi-finished product, it is necessary to reduce the quantity of flock fibers, i.e. the weight of fiber per unit of surface. Nevertheless, technological limits exist which prevent falling below a certain weight of flock fiber, due to the difficulty of controlling the distribution of the fibers on the film. In other words, when the quantity of flock fibers per unit of surface of polymer support film falls below a certain value, it is no longer possible to obtain an even distribution of the fibers, as the flocking machines do not allow effective control of the flocking process. Furthermore, when the weight is very low, the effect in terms of soft and pleasing touch of the flocking is lost or, in any case, drastically reduced.

### Objects and summary of the invention

An object of this invention is to realize a method to obtain a semi-finished flock product to use as specified above, which overcomes or reduces the limits of known methods and which drastically reduces the quantity of flock fibers per unit of surface.

According to a different aspect, an object of this invention is to provide a different type of superficial workmanship of the support or substrate, for example in polymer film or in non-woven fabric to obtain a pleasing surface effect, which prevents the uncomfortable feeling of plastic, that is the polymer film on direct contact with the skin when the support is destined to form the top sheet and/or which Is aesthetically more appreciable.

According to another aspect, an object of this invention is to provide a new semi-finished product to coat sanitary towels as well as a new type of absorbent product or item using this semi-finished product.

Therefore, the invention relates a method according to claim 1.

According to a preferred embodiment of the invention, the quantity of fibers which remains anchored to the film is between 0 and 20 g/m² and preferably between 0 and 10 g/m² and even more preferably between 0 and 5 g/m².

The adhesive can be composed for example of a polymer and cross-linkable resin. Resins suited to this use can be polymer resin of various kind and specifically, but not exclusively, vinyl resins and in particular acrylic resins, without excluding other types of resins. Resins suited to this kind of use can be the following:
- resin E821 sold by Rohm&Haas Deutschland GmbH, Germany
- LA 471 S, sold by the BASF AG, Germany
- Vinacryl resin 4333, sold by Celanese Corporation, USA.

Those listed are only some of the various resins that can be used and applied in realizing the invention.

As an alternative to the polymer cross-linkable resins it is also possible to use other adhesives, like hot melt adhesives. They are thermoplastic rubber or polyolefin based adhesives. They are sold in granules, powder or rolls, fused before or after application on the substrate. After flocking the adhesive is hardened by cooling at ambient temperature. Possible hot melt adhesives used are the following:
➢ Sanicare HM 6700; Sanicare HM 6540; Sanicare HM 521; HM 339UV, Producer Henkel KgaA, Germany
➢ ST 300, Producer Savaré I.C. srl - Specialty Adhesives, Italy
➢ XTH 81820/1; H1774; H20028UN, Producer BOSTIK FINDLEY Inc., USA
➢ Swift H 625/98, Producer FORBO Swift Adhesives, France
➢ NW1002 ZP, NW1137 ZEROPACK, Producer HB Fuller Deutschland GmbH, Germany

The flocking method according to the invention achieves a series of advantages and functions. This, In particular:
implies a three dimensional effect which, as is known, is desirable for a top sheet because it is an indication of a soft touch (guaranteed in this case also with low quantities of flock fibers), and above all because it helps the outflow of the liquid towards the lower layer:
if the support is non-woven fabric or pierced film, distribution of the flock fiber with design implies minor coverage of the holes, increasing therefore the performance of the top sheet in terms of liquid acquisition;
in distribution of the flock fiber with design, the contact surface is smaller and therefore also the rewet value will be lower compared to the case in which the flock fiber is distributed over the entire surface;
using hot polymerizable and photo hardening resins, it is possible to crosslink by UV irradiation (or microwaves, radio frequency or any IR/UV combination): in this case, once flocked, the substrate (glued and flocked) is passed through a field of ultraviolet rays to allow crosslinking of the glue, where the UV rays must strike the largest possible surface of glue and flock fiber. A solution is opted for that distributes the glue and flock fiber according to geometric or ornamental design to create more or less thin areas but in any case separated one from the others, to allow the UV rays to strike the horizontal surface and the lateral sides of the glue which traps the flock fibers: therefore moving from a full surface coating to a coating in the form of a design, the surface exposed to radiation is enlarged and crosslinking improved.

To improve adhesion of the fibers to the resin, a plasma or corona treatment can be performed on the resin itself, before being flocked; such types of treatment involve an increase to the resin polarity, increasing the ability of cohesion of the flock fibers, which are also polar. Also in this case, a print distribution of the flock fiber, involves an improvement of crosslinking as the surface exposed to the treatment increases.

Below frequent reference will be made to an anchor resin. It must be understood that this is only one of the ways of anchoring the flock fibers, which can also be fixed to the support, for example, with a hot melt adhesive or other equivalent adhesive. In this context, the term adhesive means a hot melt adhesive, a resin, or any other product that is suitable to being applied according to the criteria described and compatible with the use to which the flock material is destined.

Furthermore, specific reference will be made below to the application to a polymer film, which can also be an elastic polymer film. This is a substrate or support which makes the most of the application of the present invention, as the application of the flock fibers avoids the contact of the skin with the polymer material forming the film. Flock films are particularly ideal in the production of the back sheet of the absorbent product. Apertured flocked films are unfoldable as top sheet. It is not excluded, to use other kinds of substrate on which flocking is preformed, for example a non-woven, which may be but not necessarily apertured, preferably for the production of the top sheet.

Substantially, the invention is based on the idea of reducing the total quantity of flock fibers which remain anchored to the film applying the anchor adhesive according to a design rather than in continuous mode. In this way the overall surface of the film or other substrate or support is only partially covered with adhesive and therefore with flock fibers, reducing the quantity of raw material consumed. The distribution of the zones on which the adhesive is applied and therefore on which the fiber is anchored is such that the film has ample areas with a discontinuous but substantially even distribution of flock fibers.

While in known methods only the possibility of forming ample strip-like zones, which are completely coated with fibers, are envisaged, separated from one another by large strip-like zones which are completely free of fibers, for example in correspondence of the apertures for draining the body liquids, according to the invention the zones coated by flocked fibers have, in reality, a non continuous distribution of resin or other adhesive and therefore of fibers, i.e. a microdistribution of areas fitted with resin and fibers alternated with resin-free (and therefore fibreless) micro-surfaces.

This can be obtained for example by applying the adhesive according to a lined design or motif, i.e. including substantially parallel lines, for example in machine direction or in transversal direction or again in a direction, which is inclined by an angle different from 90° compared to the machine direction. Alternatively, the adhesive can be distributed in dots or spots or circular, ringshaped, quadrangular, polygonal areas or similar of small size and according to a dense pitch.

The overall area of the fibreless zones can vary from 1 to 99% and preferably between 10 and 90% of the overall useful area of the substrate. According to a possible embodiment, the fibreless area is equal or lower to the 50% of the useful overall area of the surface of the substrate or support. For example, it can be envisaged that the maximum distance between two fiber-coated zones is equal to or lower than 10mm and preferably between 1 and 10 mm. It is not excluded to use different distances. In particular, when the flocking has a mainly aesthetic function, the distance between flocked areas can be 5 cm. For example this can occur when flocking is used to decorate a back sheet with a logo or other way, which is a part of the finished product not destined to the direct contact with the skin of the person wearing it.

When the resin or other adhesive is applied in parallel lines, the distance between the lines can be for example equal to or lower than 10mm. The lines can have a width of between 1 and 10 mm and preferably between 1 and 5 mm and even more preferably between 1 and 2mm. Between adjacent lines an adhesive-free strip can be defined. The width of such adhesive-free strips being between 1 and 10, and preferably between 1 and 5mm and even more preferably between 2 and 3 mm.

According to a possible embodiment, the adhesive is distributed in a simple and repetitive motif, with a distance of between 1 and 10 mm. For example the resin is distributed according to discrete areas, separated by resin-free areas. This distribution can be made according to a lined design, or also in circular or similar areas. The possibility of distributing the adhesive and therefore the flock fibers in the form of a complex ornamental design, for example a floral or other design, composed by a group of areas of various form, linear or also non-linear, is also possible. In this case no distance is defined between the glued areas.

According to a different embodiment of the method according to the invention, the resin or other adhesive is distributed according to an ornamental or decorative motif, for example to define a logo or brand of the producer of the finished absorbent item on which the weblike material in flock film is applied as an external coating.

To give greater prestige and higher quality to the product, according to a possible embodiment of the method according to the invention a colored resin or other adhesive is used. Alternatively, or in combination, colored fibers can be used. For example, colored resins, in particular and preferably of a single color, and colored fibers, of a color different to that of the resin, can be applied on a white base polymer film. Or, the resin or other adhesive can be colorless or the same color of the base film, while the fibers are colored. Or, the fibers are colorless or the same color of the base film and the resin is colored. Or the film can be colored and the glue and/or the fiber can be white or colored in colors different to those of the film.

According to an advantageous embodiment of the method of this invention, the resin is applied to the film by a patterned roller. This can be a roller with a pattern realized by recesses or depressions, in which the resin is collected which is then yielded to the film. Alternatively, the resin can be applied to raised zones of the roller, and from these yielded to the film.

The fibers can have a length of between 0.35 and 2 mm and preferably between 0.4 and 0.8 mm and even more preferably between 0.5 and 0.6 mm. Alternatively, also for cost reasons, milled flock fibers can be used. In this kind of product the fiber lengths are distributed over a large area, between a minimum limit and a maximum limit, for example defined by the values indicated above.

The flock fibers can also measure between 0.3 and 3.3 dtex, and preferably between 0.3 and 1.7 dtex, and even more preferably between 0.3 and 0.95 dtex.

The fibers can be viscose, polyester, nylon®, acrylic fibers or other materials suitable and compatible with the use to which the flocked substrate is destined.

According to a possible embodiment of the invention, when a resin is used as adhesive, it can be an expandable resin. Using this kind of resin, during the crosslinking and/or polymerisation phase it expands increasing in volume, giving way to an effect of a relief design on the surface of the film. In this way a combined effect is achieved from the relief pattern, defined by the expanded resin and the flock fiber anchored by the same resin.

The expandable resin can be any resin available on the market, compatible with the type of application to which the semi-finished product obtained by flocking is destined. For example, a resin called EXPANCEL, produced by Schonox GmbH Expancel, Germany can be used.

In general the expandable resin will be a so-called self-expanding resin, that is composed of micro-spheres of thermoplastic polymers, which contain a gas (for example isobutene) or a liquid. With the temperature the fluid contained in the micro-spheres expands and the sides of it soften and deform by yielding with consequent increase in the permanent volume of the structure.

According to an improved embodiment of the invention, a perfumed substance can be applied to the material obtained. The perfumed substance can be:
1) applied by spray, immediately after flocking, before passing the film into the furnace where crosslinking and/or polymerisation of the resin occurs, or after hardening of an adhesive for example of the hot melt kind;
2) added to the adhesive before its application to the film;
3) added in the phase of activating the fibers, that is in the saline solution in which the fibers are immersed to make them polar; according to the features and the nature of the substances used.

The perfumed substances can be of various kind and typically essential oil based, like for example the essences distributed with the commercial name HS 29 by NEARCHIMICA (Italy). Alternatively perfumed microencapsulated substances can be used, composed of lipo-soluble micro-encapsulated perfumes dispersed in water. Such microencapsulated products are activated by rubbing, after which the capsules break and release the perfume. These products include for example:
- VERAROMA sold by EIGENMANN&VERONELLI SpA (Italy);
- PROFUMO MICROINCAPSULATO, sold by TILLMANNS SPA (Italia).

The fragrances can be varied; fruit-flowers, but also emollients (calendula-officinal sage-aloe).

The fragrances can be combined to cyclodextrins, to increase the release time. Cyclodextrins are cyclic oligosaccharides, used as agents for the slow release of the fragrance. In fact the perfume molecules are encapsulated in the cavities of the cyclodextrins and do not evaporate. These are held for a long period and water is necessary for the release of the perfume molecules. Practically, even small quantities of water are enough to free the perfume molecules and simultaneously the organic molecules creating the bad odors join together, going to fill the cavities previously occupied by the perfume. The cyclodextrins, therefore increase the duration of the fragrance, allowing the controlled release at the moment of real need, and at the same time act as odor-eating substances. The cyclodextrins can be applied as above by addition to the resin or other adhesive, or by spray after flocking.

The invention also concerns a web like product according to claim 28.

According to a possible embodiment of the invention, perfumes or curative substances can be applied to the product, known for their beneficial effects on the skin, like substances containing aloe, bees wax, calendula, ginseng, etc. These substances can be added directly during preparation of the resin, or sprayed on the flocked product, before it is passed to the furnace. Natural essences or extracts of plants or animal or vegetal secretions can be used. They can be soluble in water or made soluble by using tensio-active agents.

These substances, essences, extracts or active ingredients can be microencapsulated, to obtain a gradual release. During the resin preparation phase microcapsules can be added containing fragrances or active principle ingredients of the kind indicated above, with dimensions of the microcapsules of about 1 micron and resistances to temperature greater than 300°C. The process of micro-encapsulation protects the particles of substances added within the microcapsules in invisible resin which release the active substance only by light rubbing guaranteeing duration over time, when the product is packaged, while during use breakage of the micro- capsules is obtained and the consequent emission of the substances In it contained. These microcapsules are compatible with water, solvent, acrylic systems and are applicable by the most common ways of print, coating, spray and/or foulard. The microcapsules mentioned can also contain odor-eating anti-bacterial solutions or curative ingredients of various kind.

Further advantageous characteristics of the method and product according to the invention are indicated in the claims attached.

### Brief description of the drawings

The invention is better understood following the description and the drawing, which shows a practical non-limiting embodiment of the invention. More specifically the drawing shows:
Fig. 1 a scheme of the system for realizing the invention;
Fig. 2 a view of a portion of flock film in bands;
Fig. 3A-3D schematic enlargements of the surface of the film with different flocking patterns;
Fig. 4 a view of a sanitary towel produced with a flocked top sheet;
Fig. 5A, 5B and 6 schematic transversal sections according to V-V of Fig.4;
Fig. 7 and 8 schematic longitudinal sections of the flocked film in two different forms of realization;
Fig. 9 shows a scheme of the system in which the fibers are anchored by a hot melt adhesive.
Fig. 10 a schematic enlargement of a variant of the pattern according to which the flock fiber is distributed.

### Detailed description of preferred embodiment of the invention

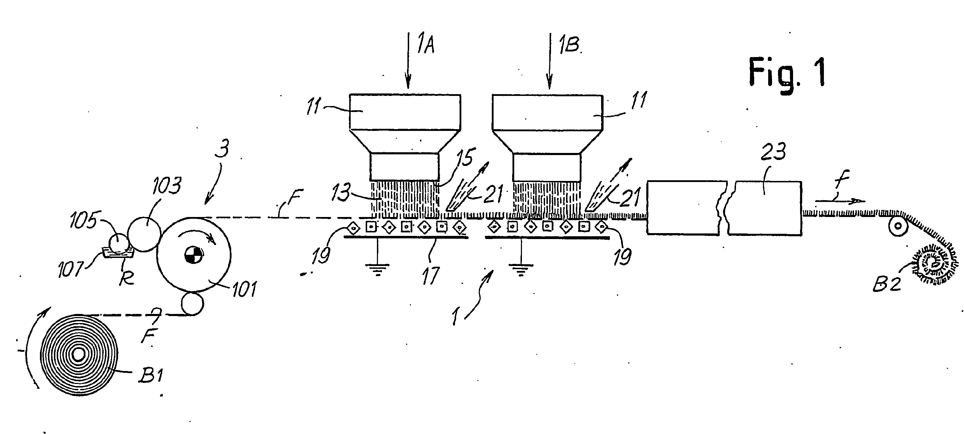

Fig. 1 shows very schematically, a system for the production of a flocked polymer film according to the invention, with the use of an adhesive with cross-linkable base resin.

B1 indicates a spool of polymer film F (or other substrate or support, for example a non-woven) to treat, which is unwound according to arrow f and fed to a flocking machine 1. Upstream of flocking machine 1 is a print application station 3 of an anchor resin, in which a polymerizable and/or cross-linkable resin R is distributed on the upper side of film F. Station 3 includes, in the example shown, a counter-roller 101 around which the polymer film F is guided. A recessed print roller 103, that is with surface recesses or depressions in which the polymerizable and/or cross-linkable resin is collected cooperates with counter-roller 101. The recesses or depressions are realized according to a preset pattern, as described below, so that resin R is applied to film F according to a corresponding pattern.

Resin R is distributed on roller 103 by a distributor roller 105, which is in surface contact with roller 103, and which picks up the polymerizable and/or cross-linkable resin R from a container, here schematically represented as a tub 109.

Polymer film F passes into the nip between rollers 101 and 103 and on its surface turned towards print roller 103 is applied resin R according to a pattern corresponding to the pattern defined by the recesses or by the depressions in roller 103. The film on which resin R has been distributed is fed to the flocking machine 1.

The flocking machine 1 shown in Fig. 1 is a double station, but the use of a flocking machine with a single station or more than two stations is also possible. Each station 1A, 1 B has a dosing device 11 from which an adjustable quantity of fiber 13 is distributed on film F below. The fall area of fibers 13 is immersed in an electrostatic field, whose lines of force are approximately at right angles to film F. The electrostatic field is obtained by two electrodes 15 and 17 laid above and below film F. In this way the fibers anchor in the layer of resin applied on the upper face of the film, directed according to the lines of force of the electrostatic field. Vibrator 19 positioned below the film keeps the latter in vibration at high frequency to allow correct distribution of the fibers and removal of the fibers which are not correctly anchored to the resin, or which fall onto the portions of surface of film F on which resin R has not been applied. The latter are sucked away by an aspirator 21.

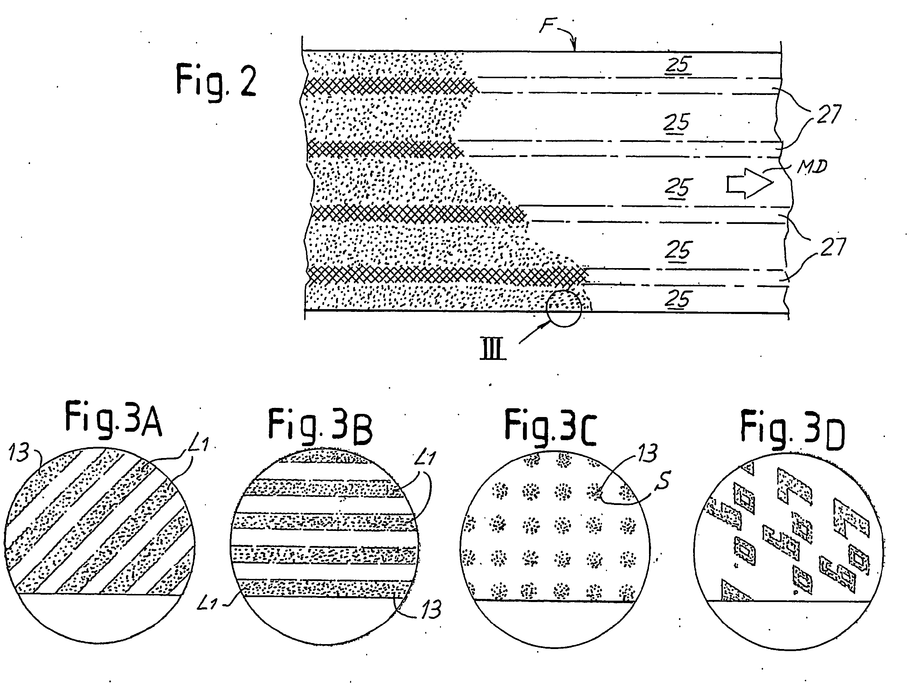

As resin R has been distributed on the surface of polymer film F according to a preset pattern, defined by the recesses of print roller 103, fibers 13 anchor on the film according to the same pattern, while the resin-free zones are also free of flocked fibers 13. Fig. 2 shows, schematically, a portion of film F in which strips or bands 25 are defined on which resin R has been applied according to a pattern, separated by bands or strips 27 completely resin free and in correspondence of which film F is apertured. Bands 27 can be realized by corresponding ring shaped zones of print roller 103 which do not receive resin R. The possibility that film F is free of apertures and/or unflocked bands is not excluded. In other words, the film can be whole and/or entirely flocked with a design.

In strips or bands 25 or on the surface of the film which is flocked resin R is distributed according to a pattern which can be for example that represented schematically in one of the Fig. 3A, 3B or 3C which are schematic enlargements of the portion indicated with III in Fig. 2

In the example of Fig. 3A in the band or strip 25 in which film F is provided with flock fibers, these are distributed according to lines or rows L1 having an inclined shape compared to the machine direction (arrow MD in Fig. 2). In the various bands 25 lines L1 can have different inclinations, in order that on film F fed between rollers 101 and 103 no forces are exerted which can make the film skid laterally during production.

In Fig. 3B the lines or rows L1 according to which the resin R has been distributed and along which by consequence are anchored fibers 13 are parallel to the machine direction MD. In a different embodiment (not shown) the rows or lines L1 could have an orthogonal direction to that indicated in Fig. 3B.

In the example of realization shown schematically in the enlargement of Fig. 3C resin R has been distributed according to spots S in which are anchored fibers 13 while in the area surrounding zones S the film is free of fiber.

As indicated in the introduction of this description the pattern according to which the resin is distributed is chosen so that the flocked areas have a sufficiently even distribution of fibers though with an extremely low weight, variable from between 0 and 30 g/m², or from 0 to 20 g/m² and preferably from 0 to 15g/m² and even more preferably from 0 to 10 g/m² and even lower than for example to between 0 and 5 g/m². According to an advantageous embodiment, the fiber is anyhow distributed with a weight greater than 0.5 g/m² and preferably greater than 1 g/m²

Distribution of the resin by way; of print roller 103 allows to realize on film F, instead of a distribution of the flock fibers 13 according to geometric patterns, also a distribution according to a decorative or ornamental pattern, writing, a logo or other to personalize the product which is obtained from this kind of production. Fig. 3D shows a solution of this kind, in which the resin has been distributed on the surface of film F by way of roller 103 according to a logo repeated on the film surface.

Resin R can be an expandable resin, which during the passage in the furnace for polymerisation and/or crosslinking (as described below) expands increasing in volume, providing a further tactile feature to the finished product. The latter will have not only superficial flocking but also an effect of superficial irregularity with more or less marked swelling in correspondence of the zones in which resin R has been applied and has subsequently expanded in phase of crosslinking.

Downstream from flocking machine 1 the film is passed into furnace 23, where resin R is crosslinked. The outgoing film is rewound in spool B2.

Film F on spool B1 can be a previously apertured, optionally three-dimensional film, or station 3, flocking machine 1 and furnace 23 can be positioned downstream of a production line section, in which a film F is apertured in continuous. In the latter case production occurs in a single line.

The possibility to aperture the film after flocking is not excluded. Nevertheless this can be problematic as the heat or the pressure applied to the film to cause the aperturing can damage the flocked fibers.

The film can be apertured on the entire surface or apertured in zones, as described in EP-A-0 598 970. If the flocked film is used to produce the back sheet of the absorbent item it is not apertured.

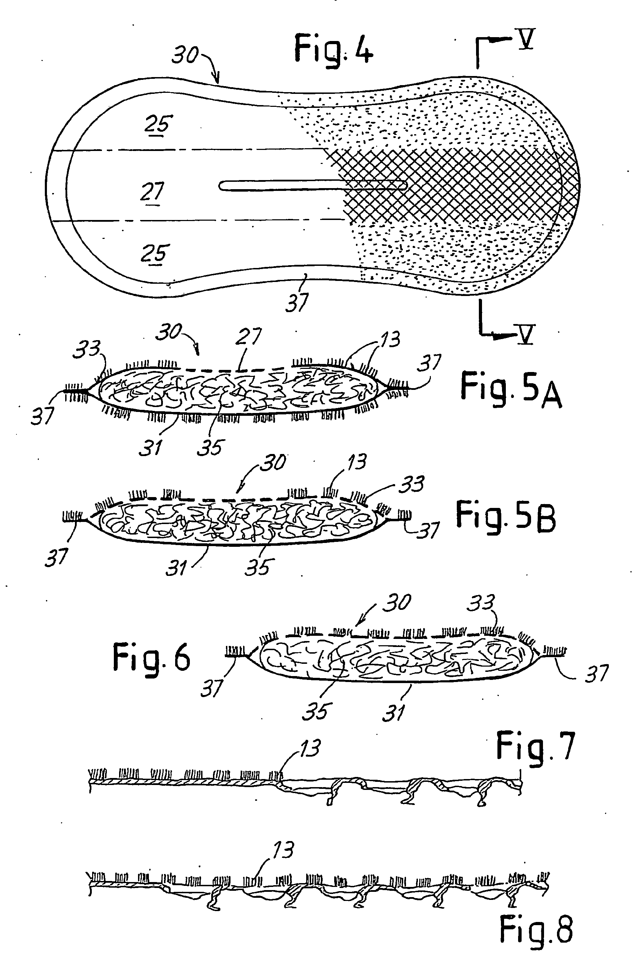

An apertured film flocked according to bands 25,27 as shown in Fig..2 can be used to produce sanitary towels, as shown in Fig. 4, 5A, 5B. 31 shows the back sheet of the towel 30 and 33 the top sheet. Absorbent material 35 is arranged between the two layers. The two layers 31 and 33 are joined along edge 37 of the towel. As is clearly shown in Fig. 4, upper layer 33 of the towel is produced with a film F of the flocked kind in bands, as shown in Fig. 2. The apertured and unflocked band 27 of film F is positioned in the central zone of towel 30, while the flocked and preferably not apertured bands 25 are arranged laterally.

In this way the plastic surface of film F is in contact with the body of the user only in the central area, while the sides have the layer of flocked fibers and therefore the skin is in contact with the fiber.

If the flock fiber is treated to be hydrophobic, this encourages the outflow of the liquid along the surface of the film towards the central zone, where it passes through the holes of the film and is quickly absorbed by the internal absorbent material. As clearly shown in Fig. 5A and 5B the film forming upper layer 33 can be apertured only in the central zone (Fig. 5A) or on the entire surface (Fig. 5B). The second solution can be preferable if the same apertured film has to be used as semi-finished product for different applications, for example sanitary towels and nappies.

The hydrophobic treatment of the flock fiber can be advantageous also in cases where it is distributed on the entire surface or in any case also on the apertured zones of the film. In fact, as the distribution of the fibers according to the invention occurs in a discrete and discontinuous manner, that is in a pattern, with small fiber-free areas alternating with or surrounding the flocked surfaces, i.e. areas with fibers, the hydrophobic quality of the fiber encourages the outflow from the flocked zones to the unflocked zones and therefore fast drainage. For example, if the fibers are distributed in rows, with transversal dimensions of the flocked and unflocked rows in the range of 1-2mm, the hydrophobic fibers encourage the flow of the liquids in the fiber free zones, which separate adjacent flocked rows.

The treatment to make the fibers hydrophobic generally occurs after the application on film F or other substrate or support, in a purposely provided station in combination with other stations, like a finishing station.

The back sheet 31 of towel 30 does not come into contact with the skin and so can be made in normal polymer film (Fig. 5B and 6). Using flock film also for the back sheet 31 (Fig. 5A) a towel with a soft touch is obtained on both sides. In Fig. 6, film F forming the top sheet is entirely flocked, rather than divided into bands 25, 27.

The polymer film can be an elastic and/or breathable film, i.e. micro-pierced, of a type known to the experts of the field.

Fig. 7 and 8 show two enlarged transversal schematic sections of a flocked film F. Fig. 7 shows a portion.of apertured film in bands and flocked in bands. It has an integral portion (not apertured) on which the flock fiber has been applied according to a pattern as described above and an apertured portion free of flock fibers. Fig. 8 instead, shows a partially apertured and partially un-apertured film , on which the fibers have been applied on the entire surface, always according to a pattern as described above, to reduce the weight of the fibers. In this case an even layer is formed of flock fibers on the entire surface of the film.

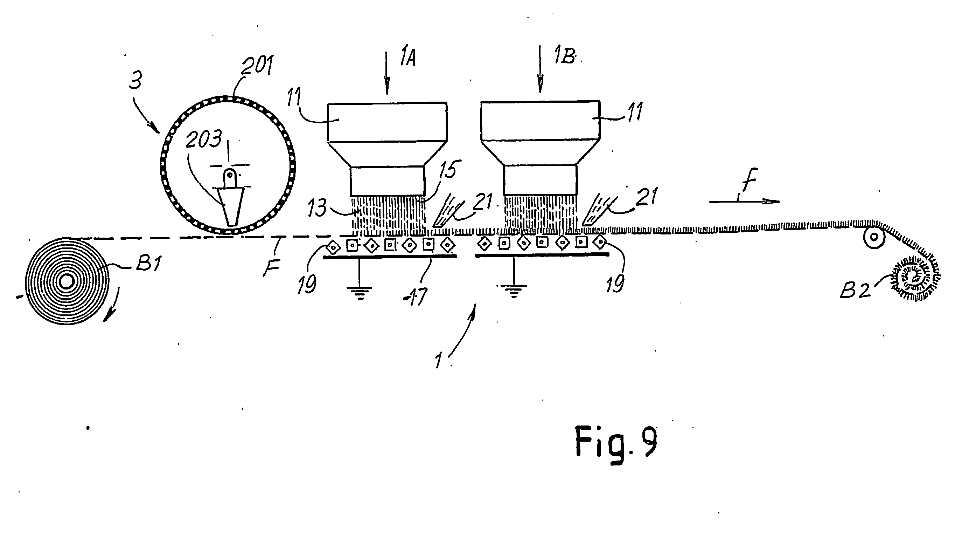

Fig. 9 shows a modified embodiment of the system. Equal numbers indicate equal or equivalent parts to those illustrated in Fig. 1 In this case the adhesive to anchor the fibers is a hot melt adhesive and a crosslinking furnace 23 is not required. The adhesive is fused and applied on film F or other substrate and hardened at ambient temperature after application of the fiber by flocking.

In the schematic example shown in Fig. 9, print application station 3 of the adhesive has a different structure and is suited to the kind of adhesive used. It includes screen cylinder 201, whose surface has holes, formed in a mesh structure, according to the pattern according to which the adhesive must be applied. Open links in the mesh allow the passage of the fused adhesive and its transfer to films F below. The fused adhesive is distributed by a hopper 203 positioned within cylinder 201.

The same kind of screen printing device can be also be used to perform the application of a cross-linkable resin.

Other kinds of devices for application of the cross-linkable resin and hot melt adhesive or other suitable adhesive are known and can be used as an alternative. For example, in the case of hot melt adhesive this can be distributed according to a pattern when it is still at its solid state, in granules or in powder and later fused when it is already on the substrate. Alternatively relief rollers can be used on whose projections is applied the fused adhesive which is then transferred to substrate F.

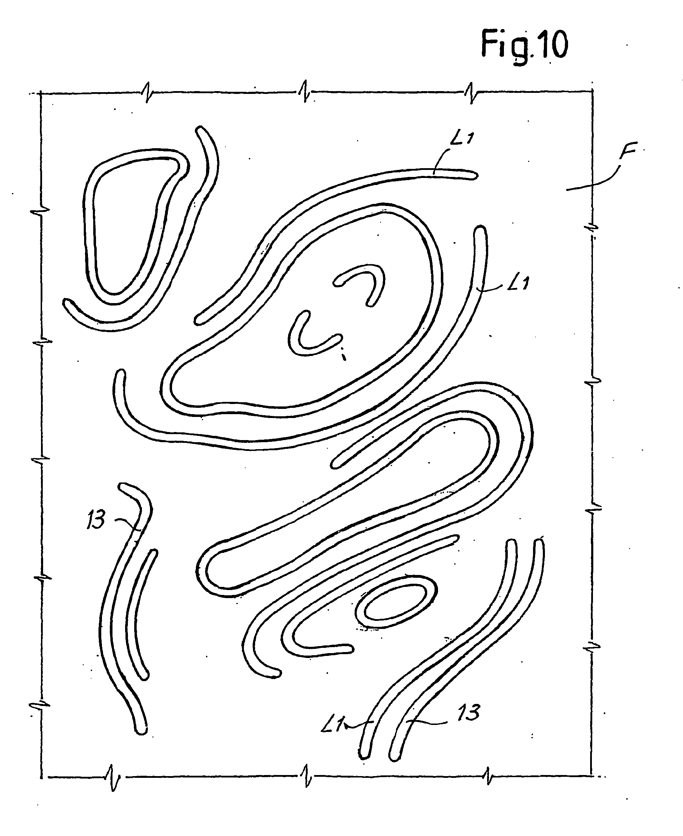

Fig. 10 shows a different pattern according to which the flock fibers can be distributed on film F. 13 indicates the flock fibers and L1 the lines of distribution of the resin and the fiber. In this case it has to do with random distribution of open or closed lines having a transversal width much smaller than the longitudinal dimension. Typically, each line L1 of resin and therefore of flocked fibers is at least 5 times longer than the width and preferably at least 10 times greater or even 15-20 times greater than the width. For example, it is possible to have lines L1 with a width of 0.5-10mm or preferably of 0.5-5mm and even more preferably of 0.5-3mm with lengths of a few centimeters or also of indefinite length that is extended for the entire material. Between contiguous lines it is possible to have empty areas, i.e. free of resin, with a distance between lines L1 for example equal to or greater than the width of the lines themselves. In this way an elevated exposition of the resin is obtained with a possible lateral irradiation with UV radiations which encourage crosslinking and/or a greater ease in treating the plasma or corona.

## Claims

1. Method for the production of a weblike material including a flocked substrate in which:
a. on at least a first side of said substrate is applied an adhesive:
b. on said first side flock fibers are distributed;
c. said fibers are anchored to the substrate by said adhesive;
**characterized in that** the quantity in weight of fibers anchored to said first side of the substrate is between 0 and 30 g/m² ; and that said adhesive is distributed in a non-continuous way on said first side of the substrate, to create zones provided with flocked fibers and zones free of flocked fibers; said adhesive being distributed according
a) to straight lines parallel to each other, between adjacent straight lines of adhesive being defined a strip free of adhesive, the width of said strips being between 1 and 10 mm and preferably between 1 and 5 mm and even more preferably between 1 and 2 mm, or
b) to a repetitive pattern formed by discrete areas, separated by areas free of adhesive, with a distance of between 1 and 10 mm and preferably between 1 mm and 5 mm, and even more preferably between 1 and 3 mm.

2. Method according to claim 1, **characterized in that** said adhesive and said fibers are distributed so that after anchorage on the substrate remains a quantity of fibers between 0 and 30 g/m² and preferably between 0 and 15 g/m² and even more preferably between 0 and 10g/m² or more preferably between 0 and 5 g/m^{2.}

3. Method according to claim 1 or 2 **characterized in that** the quantity of fibers is greater than 1 g/m² and preferably greater than 2g/m^{2.}

4. Method as claimed in claim 1 or 2 or 3, **characterized in that** the overall area of the zones free of fibers is included between 1 and 99% and preferably between 10 and 90% and even more preferably equal to or lower than 50% of the overall useful area of said first side of the substrate.

5. Method according to one or more of the preceding claims, **characterized in that** the maximum distance between two zones with fibers is equal to or less than 10mm.

6. Method according to one or more of the preceding claims, **characterized in that** said straight linesare laid out in the direction of the length of the substrate.

7. Method according to one or more of the preceding claims, **characterized in that** the distance between said straight linesis equal to or less than 10mm.

8. Method according to claims 6 or 7, **characterized in that** said straight lineshave a width of between 1 and 10 mm and preferably between 1 and 5 mm and even more preferably between 1 and 2 mm.

9. Method according to one or more of the preceding claims, **characterized in that** said adhesive is distributed according to an ornamental or decorative pattern.

10. Method according to one or more of the preceding claims, **characterized in that** said adhesive is colored.

11. Method according to one or more of the preceding claims, **characterized in that** said flock fibers are colored.

12. Method according to one or more of the preceding claims, **characterized in that** said adhesive is distributed on the substrate by a patterned roller.

13. Method according to one or more of the preceding claims, **characterized in that** said adhesive is a polymerizable and/or cross-linkable resin.

14. Method according to one or more of claims 1 to 12, **characterized in that** said adhesive is a hot melt adhesive.

15. Method according to one or more of the preceding claims, **characterized in that** said fibers have a length of between 0.35 and 2 mm and preferably between 0.4 and 0.8 mm and even more preferably between 0.5 and 0.6 mm.

16. Method according to one or more of the preceding claims, **characterized in that** said fibers are milled flock fibers.

17. Method according to one or more of the preceding claims, **characterized in that** said fibers have a count between 0.3 and 3.3 dtex, and preferably between 0.3 and 1.7 dtex, and even more preferably between 0.3 and 0.95 dtex.

18. Method according to one or more of the preceding claims, **characterized in that** said adhesive is an expandable resin, in particular a self-expanding resin.

19. Method according to one or more of the preceding claims, **characterized by** applying a perfumed substance to the substrate.

20. Method according to one or more of the preceding claims, **characterized in that** said substrate is a polymer film.

21. Method according to claim 20, **characterized in that** said polymer film is apertured.

22. Method according to one or more of claims 1 to 19, **characterized in that** said substrate is a non-woven fabric.

23. Method according to one or more of the preceding claims, **characterized by** incorporating in said adhesive at least one additive.

24. Method according to claim 23, in which said additive is selected from the group including: perfumes, curative substances, cosmetic substances, odor-eating substances, anti-bacterial substances.

25. Method according to claim 23 or 24, in which at least one of the additives is micro-encapsulated.

26. Method according to one or more of the preceding claims, in which said adhesive is applied according to lines having a smaller transversal dimension than the longitudinal dimension, preferably with a longitudinal dimension of at least 5 times greater and even more preferably at least 10 times greater than the transversal dimension.

27. Method according to claim 25, in which said lines have a width of between 0.3 and 10mm and preferably between 0.5 and 10mm, more preferably between 0.5 and 5mm and even more preferably between 0.5 and 3 mm.

28. A weblike product including a flocked substrate, **characterized in that** on at least a first side of said substrate is applied a quantity in weight of flock fibers of between 0 and 30 g/m²; and that said adhesive is distributed in a non continuous way on said first side of the substrate, to create zones with flock fibers and zones free of flock fibers; said adhesive being applied according
a) to straight lines parallel to each other; between adjacent straight lines of adhesive being defined a strip free of adhesive, the width of said strips being between 1 and 10 mm and preferably between 1 and 5 mm and even more preferably between 1 and 2 mm; said fibers being applied according to straight lines parallel to each other; or
b) to a repetitive pattern formed by discrete areas, separated by areas free of adhesive and fiber, with a distance of between 1 and 10 mm and preferably between 1 mm and 5 mm, and even more preferably between 1 and 3 mm.

29. Product according to claim 28, **characterized in that** on said first side of the substrate is applied a quantity of fibers between 0 and 30 gr/m² and preferably between 0 and 15 gr/m² and even more preferably between 0 and 10 gr/m² or more preferably between 0 and 5 gr/m².

30. Product according to claim 27 or 28, **characterized in that** the quantity of fibers is greater than 1 gr/m² and preferably greater than 2 gr/m².

31. Product according to claim 28, 29 or 30, **characterized in that** the overall area of the zones free of fibers is between 1 and 99% and preferably between 10 and 90% and even more preferably equal to or lower than 50% of the overall useful area of said first side of the substrate.

32. Product according to one or more of claims 28 to 31, **characterized in that** the maximum distance between two zones coated in fibers is equal to or less than 10mm.

33. Product according to claim 32, **characterized in that** said straight linesare laid out in the direction of the length of the substrate.

34. Product according to claim 32 or 33 **characterized in that** the distance between said straight lines is equal to or less than 10mm.

35. Product according to one or more of claims 33 to 34, **characterized in that** said straight lineshave a width of between 1 and 10 mm and preferably between 1 and 5 mm and even more preferably between 1 and 2 mm.

36. Product according to one or more of claims 28 to 35, **characterized in that** said adhesive and fibers form an ornamental or decorative pattern.

37. Product according to one or more of claims 28 to 36, **characterized in that** said adhesive is colored.

38. Product according to one or more of claims 28 to 37, **characterized in that** said flock fibers are colored.

39. Product according to one or more of claims 28 to 38, **characterized in that** said adhesive is a polymerizable and/or cross-linkable resin or a hot melt adhesive.

40. Product according to one or more of claims 28 to 39, **characterized in that** said fibers have a length of between 0.35 and 2 mm and preferably between 0.4 and 0.8 mm and even more preferably between 0.5 and 0.6 mm.

41. Product according to one or more of claims 28 to 40, **characterized in that** said fibers are milled fibers.

42. Product according to one or more of claims 28 to 41, **characterized in that** said fibers have a count between 0.3 and 3.3 dtex, and preferably between 0.3 and 1.7 dtex, and even more preferably between 0.3 and 0.95 dtex.

43. Product according to one or more of claims 28 to 42, **characterized in that** said adhesive is an expandable resin, in particular a self-expanding resin.

44. Product according to one or more of claims 28 to 43, **characterized in that** it contains a perfumed substance.

45. Product according to one or more of claims 28 to 44, **characterized in that** said substrate is a polymer film.

46. Product according to claim 45, **characterized in that** said polymer film is apertured.

47. Product according to one or more of claims 28 to 44, **characterized in that** said substrate is a non-woven.

48. Product according to one or more of claims 28 to 46, **characterized by** incorporating in said adhesive at least one additive.

49. Product according to claim 48, in which said additive is selected from the group including: perfumes, curative substances, cosmetic substances, odor-eating substances, anti-bacterial substances.

50. Product according to claim 48 or 49 in which at least one of the additives is micro-encapsulated.

51. Product according to one or more of claims 28 to 50, in which said adhesive is applied according to lines having a greater longitudinal dimension than the transversal dimension, preferably with a longitudinal dimension of at least 5 times greater and even more preferably at least 10 times greater than the transversal dimension.

52. Product according to claim 51, in which said lines have a width of between 0.3 and 10 mm and preferably between 0.5 and 10mm, more preferably between 0.5 and 5 mm and even more preferably between 0.5 and 3 mm.

## Patentansprüche

1. Verfahren zur Herstellung eines, einen beflockten Träger umfassenden, bahnförmigen Materials, wobei
a. auf wenigstens einer ersten Seite des Trägers ein Klebstoff aufgetragen wird;
b. auf dieser ersten Seite Flockfasern verteilt werden;
c. die Fasern am Trägermaterial mittels des Klebstoffes verankert werden;
**dadurch gekennzeichnet, dass** der Gewichtsanteil der auf der ersten Seite des Trägers verankerten Fasern zwischen 0 und 30 g/m² liegt; und dass der Klebstoff auf der ersten Seite des Trägers nicht durchgängig aufgetragen wird, um Abschnitte mit Flockfasern und Abschnitte frei von Flockfasern vorzusehen, wobei der Klebstoff entsprechend
a. in geraden, zueinander parallelen Linien, zwischen welchen benachbarten Linien des Klebstoffs von Klebstoff freie Streifen definiert sind, wobei die Breite der Streifen zwischen 1 und 10 mm und bevorzugt zwischen 1 und 5 mm und noch bevorzugter zwischen 1 und 2 mm liegt, oder
b. in einem sich wiederholenden Muster, das durch einzelne Bereiche geformt wird, die von klebstofffreien Bereichen in einem Abstand zwischen 1 und 10 mm und bevorzugt zwischen 1 und 5 mm und noch bevorzugter zwischen 1 und 3 mm getrennt werden, aufgetragen wird.

2. Verfahren nach Anspruch 1, **dadurch gekennzeichnet, dass** der Klebstoff und die Fasern derart verteilt werden, dass nach dem Verankern auf dem Trägers eine Fasermenge zwischen 0 und 30 g/m² und bevorzugt zwischen 0 und 15 g/m² und noch bevorzugter zwischen 0 und 10 g/m² oder bevorzugter zwischen 0 und 5 g/m² verbleibt.

3. Verfahren nach Anspruch 1 oder 2, **dadurch gekennzeichnet, dass** die Fasermenge größer als 1 g/m² und vorzugsweise größer als 2 g/m² ist.

4. Verfahren nach Anspruch 1 oder 2 oder 3, **dadurch gekennzeichnet, dass** die Gesamtfläche der Bereiche ohne Fasern innerhalb 1 und 99% liegt und bevorzugt zwischen 10 und 90% und noch bevorzugter gleich oder weniger als 50% der nutzbaren Gesamtfläche der ersten Seite des Trägers ist.

5. Verfahren nach einem oder mehreren der vorangegangenen Ansprüche, **dadurch gekennzeichnet, dass** der maximale Abstand zwischen zwei Bereichen mit Fasern kleiner oder gleich 10 mm ist.

6. Verfahren nach einem oder mehreren der vorangegangenen Ansprüche, **dadurch gekennzeichnet, dass** die geraden Linien in Längsrichtung des Trägers ausgerichtet sind.

7. Verfahren nach einem oder mehreren der vorangegangenen Ansprüche, **dadurch gekennzeichnet, dass** der Abstand zwischen den geraden Linien kleiner oder gleich 10 mm ist.

8. Verfahren nach einem der Ansprüche 6 oder 7, **dadurch gekennzeichnet, dass** die geraden Linien eine Breite zwischen 1 und 10 mm und bevorzugt zwischen 1 und 5 mm und noch bevorzugter zwischen 1 und 2 mm aufweisen.

9. Verfahren nach einem oder mehreren der vorangegangenen Ansprüche, **dadurch gekennzeichnet, dass** der Klebstoff gemäß einem ornamentalen oder verzierenden Muster verteilt wird.

10. Verfahren nach einem oder mehreren der vorangegangenen Ansprüche, **dadurch gekennzeichnet, dass** der Klebstoff färbig ist.

11. Verfahren nach einem oder mehreren der vorangegangenen Ansprüche, **dadurch gekennzeichnet, dass** die Flockfasern färbig sind.

12. Verfahren nach einem oder mehreren der vorangegangenen Ansprüche, **dadurch gekennzeichnet, dass** der Klebstoff auf dem Träger mittels einer gemusterten Walze verteilt wird.

13. Verfahren nach einem oder mehreren der vorangegangenen Ansprüche, **dadurch gekennzeichnet, dass** der Klebstoff ein polymerisierbares und/oder quervernetzbares Harz ist.

14. Verfahren nach einem der Ansprüche 1 bis 13, **dadurch gekennzeichnet, dass** der Klebstoff ein Heißschmelzkleber ist.

15. Verfahren nach einem oder mehreren der vorangegangenen Ansprüche, **dadurch gekennzeichnet, dass** die Fasern eine Länge zwischen 0,35 und 2 mm und bevorzugt zwischen 0,4 und 0,8 mm und noch bevorzugter zwischen 0,5 und 0,6 mm aufweisen.

16. Verfahren nach einem oder mehreren der vorangegangenen Ansprüche, **dadurch gekennzeichnet, dass** die Fasern gemahlene Flockfasern sind.

17. Verfahren nach einem oder mehreren der vorangegangenen Ansprüche, **dadurch gekennzeichnet, dass** die Fasern eine Zahl zwischen 0,3 und 3,3 dtex und bevorzugt zwischen 0,3 und 1,7 dtex und noch bevorzugter zwischen 0,3 und 0,95 dtex aufweisen.

18. Verfahren nach einem oder mehreren der vorangegangenen Ansprüche, **dadurch gekennzeichnet, dass** der Klebstoff ein expandierbares Harz, insbesondere ein selbstexpandierendes Harz ist.

19. Verfahren nach einem oder mehreren der vorangegangenen Ansprüche, **dadurch gekennzeichnet, dass** ein Duftstoff auf den Träger aufgetragen wird.

20. Verfahren nach einem oder mehreren der vorangegangenen Ansprüche, **dadurch gekennzeichnet, dass** der Träger ein Polymerfilm ist.

21. Verfahren nach Anspruch 20, **dadurch gekennzeichnet, dass** der Polymerfilm offen ist.

22. Verfahren nach einem der Ansprüche 1 bis 19, **dadurch gekennzeichnet, dass** der Träger ein Vlies ist.

23. Verfahren nach einem oder mehreren der vorangegangenen Ansprüche, **dadurch gekennzeichnet, dass** der Klebstoff wenigstens einen Additiv enthält.

24. Verfahren nach Anspruch 23, **dadurch gekennzeichnet, dass** das Additiv aus der Gruppe von Duftstoffen, Heilstoffen, kosmetischen Stoffen, geruchsneutralisierenden Stoffen und antibakteriellen Stoffen gewählt wird.

25. Verfahren nach Anspruch 23 oder 24, bei dem wenigstens einer der Additive mikroverkapselt ist.

26. Verfahren nach einem oder mehreren der vorangegangenen Ansprüche, **dadurch gekennzeichnet, dass** der Klebstoff entsprechend Linien aufgetragen wird, deren Ausdehnung in Querrichtung kleiner ist als deren Ausdehnung in Längsrichtung, bevorzugt mit einer Ausdehnung in Längsrichtung, die 5 mal größer und noch bevorzugter 10 mal größer ist als die Ausdehnung in Querrichtung.

27. Verfahren nach Anspruch 25, wobei die geraden Linien eine Breite zwischen 0,3 und 10 mm und bevorzugt zwischen 0,5 und 10 mm und bevorzugter zwischen 0,5 und 5 mm oder noch bevorzugter zwischen 0,5 und 3 mm aufweisen.

28. Bahnförmiges Erzeugnis mit einem beflockten Träger, **dadurch gekennzeichnet, dass** die Gewichtsanteil der auf einer ersten Seite des Trägers aufgebrachten Flockfasern zwischen 0 und 30 g/m² liegt; und dass der Klebstoff auf der ersten Seite des Trägers nicht durchgängig aufgetragen ist, um Abschnitte mit Flockfasern und Abschnitte frei von Flockfasern zu erzeugen, wobei der Klebstoff wobei der Klebstoff entsprechend
a. in geraden, zueinander parallelen Linien, zwischen welchen benachbarten Linien des Klebstoffs von Klebstoff freie Streifen definiert sind, wobei die Breite der Streifen zwischen 1 und 10 mm und bevorzugt zwischen 1 und 5 mm und noch bevorzugter zwischen 1 und 2 mm liegt; wobei die Fasern entsprechend zueinander paralleler Linien aufgebracht sind;
oder
b. in einem sich wiederholenden Muster, das durch einzelne Bereiche geformt wird, die von klebstofffreien Bereichen in einem Abstand zwischen 1 und 10 mm und bevorzugt zwischen 1 und 5 mm und noch bevorzugter zwischen 1 und 3 mm getrennt werden,
aufgetragen ist.

29. Erzeugnis nach Anspruch 28, **dadurch gekennzeichnet, dass** auf der ersten Seite des Trägers ein Fasermenge zwischen 0 und 30 g/m² und bevorzugt zwischen 0 und 15 g/m² und noch bevorzugter zwischen 0 und 10 g/m² oder bevorzugter zwischen 0 und 5 g/m² aufgetragen ist.

30. Erzeugnis nach Anspruch 27 oder 28, **dadurch gekennzeichnet, dass** die Fasermenge größer als 1 g/m² und vorzugsweise größer als 2 g/m² ist.

31. Erzeugnis nach Anspruch 28, 29 oder 30, **dadurch gekennzeichnet, dass** die Gesamtfläche der von Fasern freien Bereiche zwischen 1 und 99% liegt und bevorzugt zwischen 10 und 90% und noch bevorzugter gleich oder weniger als 50% der nutzbaren Gesamtfläche der ersten Seite des Trägers ist.

32. Erzeugnis nach einem oder mehreren der Ansprüche 28 bis 31, **dadurch gekennzeichnet, dass** der maximale Abstand zwischen zwei mit Fasern bedeckten Bereichen kleiner oder gleich 10 mm ist.

33. Erzeugnis nach Anspruch 32, **dadurch gekennzeichnet, dass** die geraden Linien in Längsrichtung des Trägers ausgerichtet sind.

34. Erzeugnis nach Anspruch 32 oder 33, **dadurch gekennzeichnet, dass** der Abstand zwischen den geraden Linien kleiner oder gleich 10 mm ist.

35. Erzeugnis nach einem der Ansprüche 33 bis 34, **dadurch gekennzeichnet, dass** die geraden Linien eine Breite zwischen 1 und 10 mm und bevorzugt zwischen 1 und 5 mm und noch bevorzugter zwischen 1 und 2 mm aufweisen.

36. Erzeugnis nach einem oder mehreren der Ansprüche 28 bis 35, **dadurch gekennzeichnet, dass** der Klebstoff und die Fasern ein ornamentales oder verzierendes Muster bilden.

37. Erzeugnis nach einem oder mehreren der Ansprüche 28 bis 36, **dadurch gekennzeichnet, dass** der Klebstoff färbig ist.

38. Erzeugnis nach einem oder mehr der Ansprüche 28 bis 37, **dadurch gekennzeichnet, dass** die Flockfasern färbig sind.

39. Erzeugnis nach einem oder mehreren der Ansprüche 28 bis 38, **dadurch gekennzeichnet, dass** der Klebstoff ein polymerisierbares und/oder quervernetzbares Harz oder ein Heißschmelzkleber ist.

40. Erzeugnis nach einem oder mehreren der Ansprüche 28 bis 39, **dadurch gekennzeichnet, dass** die Fasern eine Länge zwischen 0,35 und 2 mm und bevorzugt zwischen 0,4 und 0,8 mm und noch bevorzugter zwischen 0,5 und 0,6 mm aufweisen.

41. Erzeugnis nach einem oder mehreren der Ansprüche 28 bis 40, **dadurch gekennzeichnet, dass** die Fasern gemahlene Fasern sind.

42. Erzeugnis nach einem oder mehreren der Ansprüche 28 bis 41, **dadurch gekennzeichnet, dass** die Fasern eine Zahl zwischen 0,3 und 3,3 dtex und bevorzugt zwischen 0,3 und 1,7 dtex und noch bevorzugter zwischen 0,3 und 0,95 dtex aufweisen.

43. Erzeugnis nach einem oder mehreren der Ansprüche 28 bis 42, **dadurch gekennzeichnet, dass** der Klebstoff ein expandierbares Harz, insbesondere ein selbstexpandierendes Harz ist.

44. Erzeugnis nach einem oder mehreren der Ansprüche 28 bis 43, **dadurch gekennzeichnet, dass** es einen Duftstoff enthält.

45. Erzeugnis nach einem oder mehreren der Ansprüche 28 bis 44, **dadurch gekennzeichnet, dass** der Träger ein Polymerfilm ist.

46. Erzeugnis nach Anspruch 45, **dadurch gekennzeichnet, dass** der Polymerfilm offen ist.

47. Erzeugnis nach einem der Ansprüche 28 bis 44, **dadurch gekennzeichnet, dass** das Trägermaterial ein Vlies ist.

48. Erzeugnis nach einem oder mehreren der Ansprüche 28 bis 46, **dadurch gekennzeichnet, dass** der Klebstoff wenigstens ein Additiv enthält.

49. Erzeugnis nach Anspruch 48, wobei das Additiv aus der Gruppe von Duftstoffen, Heilstoffen, kosmetischen Stoffen, geruchsneutralisierenden Stoffen und antibakteriellen Stoffen gewählt ist.

50. Erzeugnis nach Anspruch 48 oder 49, bei dem wenigstens einer der Additive mikroverkapselt ist.

51. Erzeugnis nach einem oder mehreren der Ansprüche 28 bis 50, wobei der Klebstoff in Linien aufgetragen ist, deren Ausdehnung in Querrichtung kleiner ist als deren Ausdehnung in Längsrichtung, bevorzugt mit einer Ausdehnung in Längsrichtung, die 5 mal größer und noch bevorzugter 10 mal größer ist als die Ausdehnung in Querrichtung.

52. Erzeugnis nach Anspruch 51, wobei die Linien eine Breite zwischen 0,3 und 10 mm und bevorzugt zwischen 0,5 und 10 mm und bevorzugter zwischen 0,5 und 5 mm oder noch bevorzugter zwischen 0,5 und 3 mm aufweisen.

## Revendications

1. Procédé pour la production d'un matériau en bande comprenant un substrat floqué dans lequel :
a. sur au moins un premier côté dudit substrat est appliqué un adhésif ;
b. sur ledit premier côté sont distribuées des fibres de flocage ;
c. lesdites fibres sont ancrées au substrat par ledit adhésif ;
**caractérisé en ce que** la quantité en poids de fibres ancrées audit premier côté du substrat est comprise entre 0 et 30 g/m² ; et **en ce que** ledit adhésif est distribué d'une façon non continue sur ledit premier côté du substrat, de façon à créer des zones pourvues de fibres floquées et des zones dépourvues de fibres floquées ; ledit adhésif étant distribué selon
a) des lignes droites parallèles les unes aux autres, entre des lignes droites adjacentes d'adhésif qui sont définies comme une bande dépourvue d'adhésif, la largeur desdites bandes étant comprise entre 1 et 10 mm et de préférence entre 1 et 5 mm et encore plus préférablement entre 1 et 2 mm, ou
b) un motif répétitif formé de zones distinctes, séparées par des zones dépourvues d'adhésif, avec une distance comprise entre 1 et 10 mm et de préférence entre 1 mm et 5 mm, et encore plus préférablement entre 1 et 3 mm.

2. Procédé selon la revendication 1, **caractérisé en ce que** ledit adhésif et lesdites fibres sont distribués de sorte qu'après ancrage sur le substrat, il reste une quantité de fibres comprise entre 0 et 30 g/m² et de préférence entre 0 et 15 g/m² et encore plus préférablement entre 0 et 10 g/m² ou plus préférablement entre 0 et 5 g/m².

3. Procédé selon la revendication 1 ou 2, **caractérisé en ce que** la quantité de fibres est supérieure à 1 g/m² et de préférence supérieure à 2 g/m²

4. Procédé selon la revendication 1 ou 2 ou 3, **caractérisé en ce que** l'aire globale des zones dépourvues de fibres est comprise entre 1 et 99 % et de préférence entre 10 et 90 % et encore plus préférablement égale ou inférieure à 50 % de l'aire utile globale dudit premier côté du substrat.

5. Procédé selon une ou plusieurs des revendications précédentes, **caractérisé en ce que** la distance maximale entre deux zones avec fibres est égale ou inférieure à 10 mm.

6. Procédé selon une ou plusieurs des revendications précédentes, **caractérisé en ce que** lesdites lignes droites sont disposées dans le sens de la longueur du substrat.

7. Procédé selon une ou plusieurs des revendications précédentes, **caractérisé en ce que** la distance entre lesdites lignes droites est égale ou inférieure à 10 mm.

8. Procédé selon les revendications 6 ou 7, **caractérisé en ce que** lesdites lignes droites ont une largeur comprise entre 1 et 10 mm et de préférence entre 1 et 5 mm et encore plus préférablement entre 1 et 2 mm.

9. Procédé selon une ou plusieurs des revendications précédentes, **caractérisé en ce que** ledit adhésif est distribué selon un motif ornemental ou décoratif.

10. Procédé selon une ou plusieurs des revendications précédentes, **caractérisé en ce que** ledit adhésif est coloré.

11. Procédé selon une ou plusieurs des revendications précédentes, **caractérisé en ce que** lesdites fibres de flocage sont colorées.

12. Procédé selon une ou plusieurs des revendications précédentes, **caractérisé en ce que** ledit adhésif est distribué sur le substrat par un rouleau à motif.

13. Procédé selon une ou plusieurs des revendications précédentes, **caractérisé en ce que** ledit adhésif est une résine polymérisable et/ou réticulable.

14. Procédé selon une ou plusieurs des revendications 1 à 12, **caractérisé en ce que** ledit adhésif est un adhésif thermofusible.

15. Procédé selon une ou plusieurs des revendications précédentes, **caractérisé en ce que** lesdites fibres ont une longueur comprise entre 0,35 et 2 mm et de préférence entre 0,4 et 0,8 mm et encore plus préférablement entre 0,5 et 0,6 mm.

16. Procédé selon une ou plusieurs des revendications précédentes, **caractérisé en ce que** lesdites fibres sont des fibres de flocage broyées.

17. Procédé selon une ou plusieurs des revendications précédentes, **caractérisé en ce que** lesdites fibres ont un titrage compris entre 0,3 et 3,3 dtex, et de préférence entre 0,3 et 1,7 dtex, et encore plus préférablement entre 0,3 et 0,95 dtex.

18. Procédé selon une ou plusieurs des revendications précédentes, **caractérisé en ce que** ledit adhésif est une résine expansible, en particulier une résine auto-expansible.

19. Procédé selon une ou plusieurs des revendications précédentes, **caractérisé par** l'application d'une substance parfumée au substrat.

20. Procédé selon une ou plusieurs des revendications précédentes, **caractérisé en ce que** ledit substrat est un film polymère.

21. Procédé selon la revendication 20, **caractérisé en ce que** ledit film polymère est perforé.

22. Procédé selon une ou plusieurs des revendications 1 à 19, **caractérisé en ce que** ledit substrat est une étoffe non tissée.

23. Procédé selon une ou plusieurs des revendications précédentes, **caractérisé par** l'incorporation dans ledit adhésif d'au moins un additif.

24. Procédé selon la revendication 23, dans lequel ledit additif est choisi dans le groupe comprenant : des parfums, des substances de type durcisseur, des substances cosmétiques, des substances déodorantes, des substances antibactériennes.

25. Procédé selon la revendication 23 ou 24, dans lequel au moins un des additifs est micro-encapsulé.

26. Procédé selon une ou plusieurs des revendications précédentes, dans lequel ledit adhésif est appliqué selon des lignes ayant une dimension transversale plus petite que la dimension longitudinale, de préférence avec une dimension longitudinale d'au moins 5 fois plus grande et encore plus préférablement au moins 10 fois plus grande que la dimension transversale.

27. Procédé selon la revendication 25, dans lequel lesdites lignes ont une largeur comprise entre 0,3 et 10 mm et de préférence entre 0,5 et 10 mm, plus préférablement entre 0,5 et 5 mm et encore plus préférablement entre 0,5 et 3 mm.

28. Produit en bande comprenant un substrat floqué, **caractérisé en ce que** sur au moins un premier côté dudit substrat est appliquée une quantité en poids de fibres de flocage comprise entre 0 et 30 g/m² ; et **en ce que** ledit adhésif est distribué d'une façon non continue sur ledit premier côté du substrat, de façon à créer des zones avec des fibres de flocage et des zones dépourvues de fibres de flocage ; ledit adhésif étant appliqué selon
a) des lignes droites parallèles les unes aux autres ; entre des lignes droites adjacentes d'adhésif qui sont définies comme une bande dépourvue d'adhésif, la largeur desdites bandes étant comprise entre 1 et 10 mm et de préférence entre 1 et 5 mm et encore plus préférablement entre 1 et 2 mm, ou lesdites fibres étant appliqués selon des lignes droites parallèles les unes aux autres ;
ou
b) un motif répétitif formé de zones distinctes, séparées par des zones dépourvues d'adhésif et de fibre, avec une distance comprise entre 1 et 10 mm et de préférence entre 1 mm et 5 mm, et encore plus préférablement entre 1 et 3 mm.

29. Produit selon la revendication 28, **caractérisé en ce que** sur ledit premier côté du substrat est appliquée une quantité de fibres comprise entre 0 et 30 g/m² et de préférence entre 0 et 15 g/m² et encore plus préférablement entre 0 et 10 g/m² ou plus préférablement entre 0 et 5 g/m².

30. Produit selon la revendication 27 ou 28, **caractérisé en ce que** la quantité de fibres est supérieure à 1 g/m² et de préférence supérieure à 2 g/m²

31. Produit selon la revendication 28, 29 ou 30, **caractérisé en ce que** l'aire globale des zones dépourvues de fibres est comprise entre 1 et 99 % et de préférence entre 10 et 90 % et encore plus préférablement égale ou inférieure à 50 % de l'aire utile globale dudit premier côté du substrat.

32. Produit selon une ou plusieurs des revendications 28 à 31, **caractérisé en ce que** la distance maximale entre deux zones revêtues en fibres est égale ou inférieure à 10 mm.

33. Produit selon la revendication 32, **caractérisé en ce que** lesdites lignes droites sont disposées dans le sens de la longueur du substrat.

34. Produit selon la revendication 32 ou 33, **caractérisé en ce que** la distance entre lesdites lignes droites est égale ou inférieure à 10 mm.

35. Produit selon une ou plusieurs des revendications 33 à 34, **caractérisé en ce que** lesdites lignes droites ont une largeur comprise entre 1 et 10 mm et de préférence entre 1 et 5 mm et encore plus préférablement entre 1 et 2 mm.

36. Produit selon une ou plusieurs des revendications 28 à 35, **caractérisé en ce que** ledit adhésif et lesdites fibres forment un motif ornemental ou décoratif.

37. Produit selon une ou plusieurs des revendications 28 à 36, **caractérisé en ce que** ledit adhésif est coloré.

38. Produit selon une ou plusieurs des revendications 28 à 37, **caractérisé en ce que** lesdites fibres de flocage sont colorées.

39. Produit selon une ou plusieurs des revendications 28 à 38, **caractérisé en ce que** ledit adhésif est une résine polymérisable et/ou réticulable ou un adhésif thermofusible.

40. Produit selon une ou plusieurs des revendications 28 à 39, **caractérisé en ce que** lesdites fibres ont une longueur comprise entre 0,35 et 2 mm et de préférence entre 0,4 et 0,8 mm et encore plus préférablement entre 0,5 et 0,6 mm.

41. Produit selon une ou plusieurs des revendications 28 à 40, **caractérisé en ce que** lesdites fibres sont des fibres de flocage broyées.

42. Produit selon une ou plusieurs des revendications 28 à 41, **caractérisé en ce que** lesdites fibres ont un titrage compris entre 0,3 et 3,3 dtex, et de préférence entre 0,3 et 1,7 dtex, et encore plus préférablement entre 0,3 et 0,95 dtex

43. Produit selon une ou plusieurs des revendications 28 à 42, **caractérisé en ce que** ledit adhésif est une résine expansible, en particulier une résine auto-expansible.

44. Produit selon une ou plusieurs des revendications 28 à 43, **caractérisé en ce qu'**il contient une substance parfumée.

45. Produit selon une ou plusieurs des revendications 28 à 44, **caractérisé en ce que** ledit substrat est un film polymère.

46. Produit selon la revendication 45, **caractérisé en ce que** ledit film polymère est perforé.

47. Produit selon une ou plusieurs des revendications 28 à 44, **caractérisé en ce que** ledit substrat est un non-tissé.

48. Produit selon une ou plusieurs des revendications 28 à 46, **caractérisé par** l'incorporation dans ledit adhésif d'au moins un additif.

49. Produit selon la revendication 48, dans lequel ledit additif est choisi dans le groupe comprenant : des parfums, des substances de type durcisseur, des substances cosmétiques, des substances déodorantes, des substances antibactériennes.

50. Produit selon la revendication 48 ou 49, dans lequel au moins un des additifs est micro-encapsulé.

51. Produit selon une ou plusieurs des revendications 28 à 50, dans lequel ledit adhésif est appliqué selon des lignes une dimension longitudinale plus grande que la dimension transversale, de préférence avec une dimension longitudinale d'au moins 5 fois plus grande et encore plus préférablement au moins 10 fois plus grande que la dimension transversale.

52. Produit selon la revendication 51, dans lequel lesdites lignes ont une largeur comprise entre 0,3 et 10 mm et de préférence entre 0,5 et 10 mm, plus préférablement entre 0,5 et 5 mm et encore plus préférablement entre 0,5 et 3 mm.
